(19) Europäisches Patentamt
European Patent Office
Office européen des brevets

(11) **EP 4 447 144 A1**

(12) **EUROPEAN PATENT APPLICATION**

(43) Date of publication:
**16.10.2024 Bulletin 2024/42**

(21) Application number: **24166255.0**

(22) Date of filing: **26.03.2024**

(51) International Patent Classification (IPC):
***H01M 4/133*** *(2010.01)* ***H01M 4/587*** *(2010.01)*
***H01M 10/0525*** *(2010.01)* ***H01M 10/0567*** *(2010.01)*
*H01M 4/02* *(2006.01)*

(52) Cooperative Patent Classification (CPC):
**H01M 10/0525; H01M 4/133; H01M 4/587;**
**H01M 10/0567;** H01M 2004/021; H01M 2004/027;
H01M 2300/0025; Y02E 60/10

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 CN 202310322545**

(71) Applicant: **Ningde Amperex Technology Limited**
**Ningde, Fujian 352100 (CN)**

(72) Inventors:
- **LIU, Junfei**
**Ningde City, Fujian Province, People s Republic of China, 352100 (CN)**
- **CUI, Hui**
**Ningde City, Fujian Province, People s Republic of China, 352100 (CN)**
- **ZHENG, Yezhen**
**Ningde City, Fujian Province, People s Republic of China, 352100 (CN)**
- **LI, Wenqiang**
**Ningde City, Fujian Province, People s Republic of China, 352100 (CN)**

(74) Representative: **Icosa**
**83 avenue Denfert-Rochereau**
**75014 Paris (FR)**

(54) **ELECTROCHEMICAL DEVICE AND ELECTRONIC DEVICE CONTAINING SAME**

(57) An electrochemical device includes a negative electrode and an electrolyte solution. The negative electrode includes a negative current collector and a negative electrode mixture layer disposed on a surface of the negative current collector. The negative electrode mixture layer includes a first section, a second section, and a third section arranged sequentially in a thickness direction. The first section, the second section, and the third section are of an identical thickness. A ratio of a porosity P1 of the first section to a porosity P2 of the third section is P. The electrolyte solution includes an additive A. The additive A is at least one selected from a compound of Formula I or a compound of Formula II (the groups in Formula I and Formula II are defined in the specification):

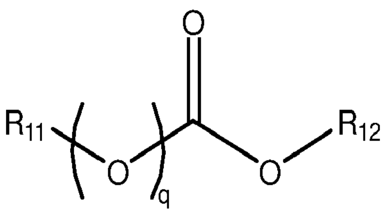 Formula I 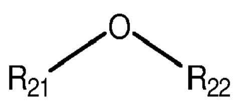 Formula II.

Based on a total mass of the electrolyte solution, a mass percent of the additive A is w%, satisfying $1 \leq w \leq 60$, $P = P1/P2$, and $P/(w\% + 1) \geq 50\%$.

EP 4 447 144 A1

## Description

### TECHNICAL FIELD

This application relates to the technical field of energy storage, and in particular, to an electrochemical device and an electronic device containing same.

### BACKGROUND

Due to advantages such as a high energy density, a high operating voltage plateau, a low self-discharge rate, longevity, and environmental friendliness, electrochemical devices (such as a lithium-ion battery) have been applied in a wide range of fields and industries such as consumer products (for example, a mobile phone, a laptop computer, and a camera), energy storage products (for example, household energy storage, energy storage station, and UPS power supply), and new energy vehicles. With the continuous diversification of the application scenarios of products, the requirement on the energy density of the battery is increasingly higher. For the purpose of improving the energy density, the processing technology of electrode plates in addition to a high gravimetric capacity of the material plays a key role. A current method for increasing the amount of active material carried on an electrode plate is to perform a pressure treatment on the electrode plate. However, during the pressure treatment in such a method, the applied force is non-uniform and may lead to a change in the porosity of the electrode plate in a thickness direction. When the porosity of an upper layer of the electrode plate is relatively large, the active material tends to form a positive concentration gradient and facilitates transport of lithium ions inside the electrode plate. However, when a electrolyte solution fails to infiltrate in time a lower layer of the electrode plate at which the porosity is relatively how, the transport of the lithium ions will be slowed down, thereby ultimately affecting the performance of the battery adversely.

Therefore, an electrochemical device needs to be developed in this field to achieve a large amount of active material carried on the electrode plate without deteriorating the transport of lithium ions due to gradient change of the porosity.

### SUMMARY

According to a first aspect of this application, this application provides an electrochemical device. The electrochemical device includes a negative electrode and an electrolyte solution. The negative electrode includes a negative current collector and a negative electrode mixture layer disposed on at least one surface of the negative current collector. The negative electrode mixture layer includes a first section, a second section, and a third section arranged sequentially in a thickness direction. The third section is contiguous to the negative current collector. The second section is arranged between the first section and the third section in the thickness direction. The first section, the second section, and the third section are of an identical thickness. A ratio of a porosity P1 of the first section to a porosity P2 of the third section is P.

The electrolyte solution includes an additive A, and the additive A is at least one selected from a compound of Formula I or a compound of Formula II:

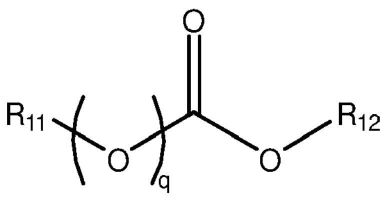
Formula I

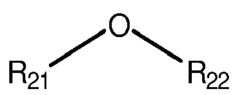
Formula II

In the formulas above, q is 0 or 1, at least one of $R_{11}$ or $R_{12}$ is selected from fluorine-substituted $C_1$ to $C_3$ alkyls, and $R_{21}$ and $R_{22}$ are selected from fluorine-substituted or unsubstituted $C_1$ to $C_4$ alkyls.

Based on a total mass of the electrolyte solution, a mass percent of the additive A is w%, satisfying $1 \leq w \leq 60$.

The electrochemical device satisfies the following conditions: P = P1/P2, and P/(w% + 1) ≥ 50%. By introducing an appropriate amount of additive A of a relatively low surface tension into the electrolyte solution, this application can reduce the surface tension of the electrolyte solution, accelerate infiltration of the electrolyte solution, and ensure that the electrolyte solution is distributed sufficiently and uniformly in regions of different porosities on the negative electrode mixture layer, thereby reducing polarization during charging of the electrochemical device and enhancing the charging capabilities.

In some embodiments, the compound of Formula I includes at least one of the following compounds:

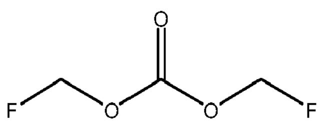
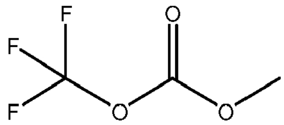
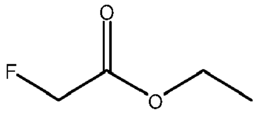
,

Formula I-1 Formula I-2 Formula I-3

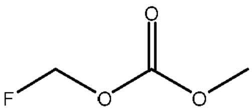
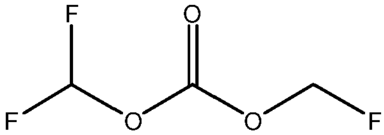
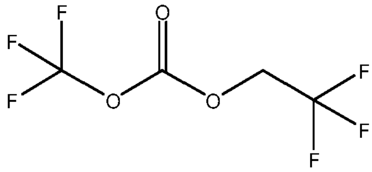
,

Formula I-4 Formula I-5 Formula I-6

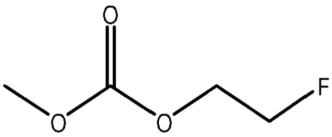
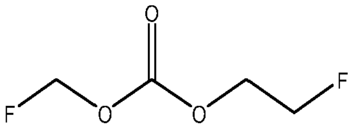
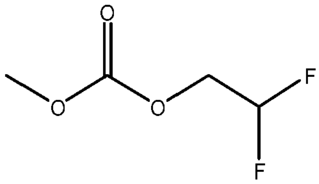
,

Formula I-7 Formula I-8 Formula I-9

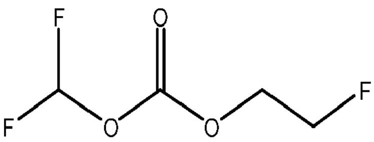
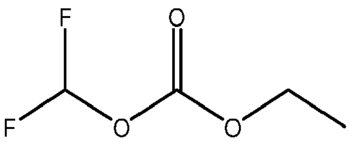
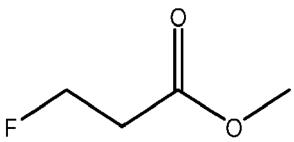
,

Formula I-10 Formula I-11 Formula I-12

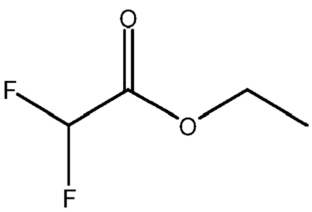
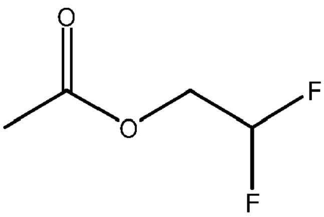
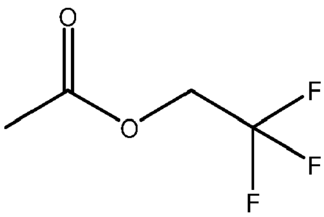
,

Formula I-13 Formula I-14 Formula I-15

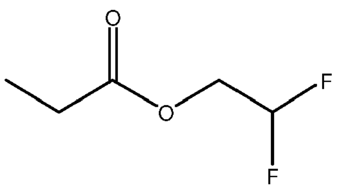 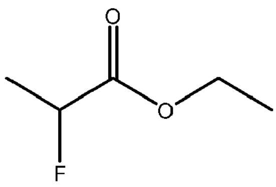 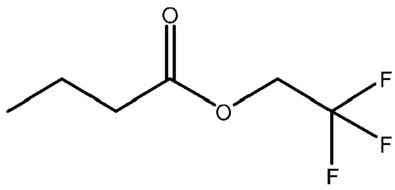 ,

Formula I-16 Formula I-17 Formula I-18

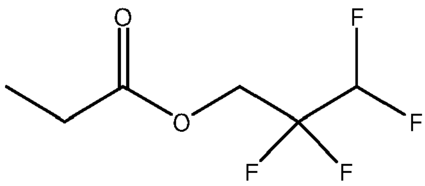 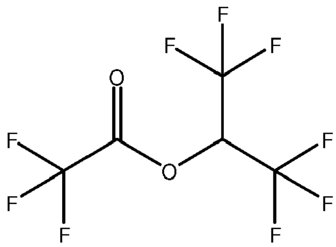 ;

Formula I-19 Formula I-20

and/or,

the compound of Formula II includes at least one of the following compounds:

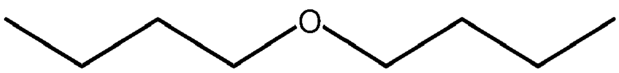 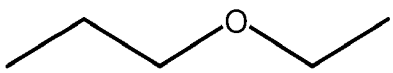 ,

Formula II-1 Formula II-2

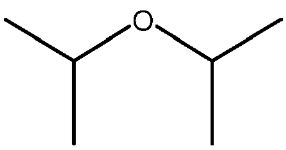 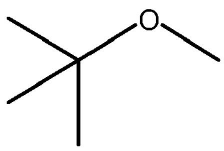 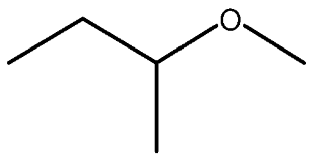 ,

Formula II-3 Formula II-4 Formula II-5

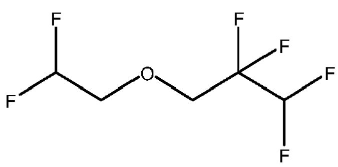 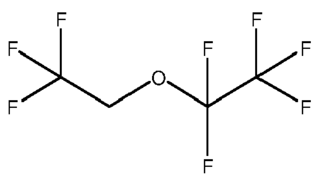 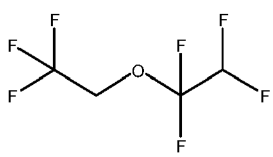 ,

Formula II-6 Formula II-7 Formula II-8

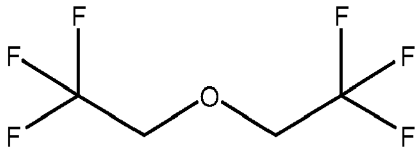 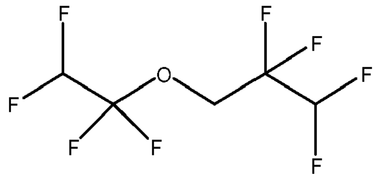.

Formula II-9 Formula II-10

In some embodiments, $3 \le w \le 50$.

In some embodiments, $60\% \le P/(w\% + 1) \le 330\%$.

In some embodiments, $3 \le w \le 45$.

In some embodiments, $60\% \le P/(w\% + 1) \le 230\%$.

In some embodiments, $P1 \ge 0.8P2$.

In some embodiments, P1 is 20% to 50%.

In some embodiments, the electrolyte solution further includes an additive B. The additive B is at least one selected from a compound of Formula III or a nitrile additive:

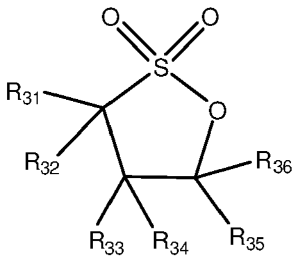 Formula III.

In Formula III above, $R_{31}$, $R_{32}$, $R_{33}$, $R_{34}$, $R_{35}$, and $R_{36}$ each are independently selected from H, a halogen atom, a halogen-substituted or unsubstituted C1 to C6 alkyl, or a C2 to C6 alkenyl or alkynyl.

The nitrile additive includes at least one of succinonitrile, glutaronitrile, adiponitrile, pimelonitrile, suberonitrile, sebaconitrile, azelonitrile, methyl glutaronitrile, 1,3,6-hexanetricarbonitrile, or trans-hexene dinitrile.

Based on the total mass of the electrolyte solution, a mass percent of the additive B is a%, satisfying $0.1 \le a \le 6$.

In some embodiments, the compound of Formula III includes at least one of the following compounds:

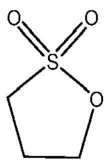 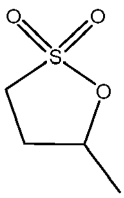 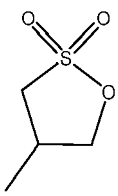 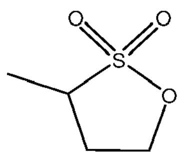 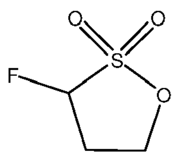 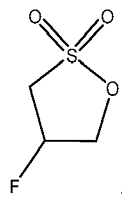,

Formula III-1 Formula III-2 Formula III-3 Formula III-4 Formula III-5 Formula III-6

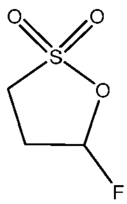 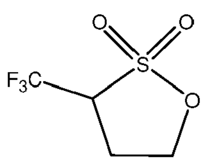 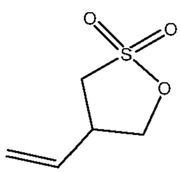 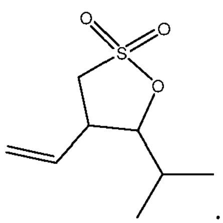.

Formula III-7 Formula III-8 Formula III-9 Formula III-10

In some embodiments, the electrolyte solution further includes an additive C. A hydrophilic-lipophilic balance value of the additive C is 0 to 15. Based on the total mass of the electrolyte solution, a mass percent of the additive C is b%, satisfying: $0.01 \leq b \leq 1$.

In some embodiments, the additive C includes at least one of the following compounds:

C1: $C_8F_{17}$-$SO_2N(C_3H_7)$-$(CH_2CH_2)_{20}$-$SO_3Li$;
C2: lithium perfluorooctyl(trifluoromethyl-sulfonyl)imide;
C3: lithium bis(nonafluorobutyl-sulfonyl)imide;
C4: lithium (nonafluorobutyl-trifluoromethyl-sulfonyl)imide;
C5: poly(oxyethylene)$_8$octylphenyl ether HO-$(CH_2CH_2O)_8$-Ph-$C_8H_{17}$; or
C6: poly(oxyethylene)$_{20}$octadecyl ether HO-$(CH_2CH_2O)_{20}$-O-$C_{18}H_{37}$.

In some embodiments, a true density TD1 of the first section and a true density TD2 of the third section satisfy: $TD1 \leq TD2$.

In some embodiments, the true density TD1 of the first section and the true density TD2 of the third section satisfy: TD1 = TD2, and the first section and the third section are of identical composition.

According to a second aspect of this application, this application provides an electronic device. The electronic device includes the electrochemical device disclosed in the first aspect of this application.

## BRIEF DESCRIPTION OF DRAWINGS

Figure shows is a schematic diagram of an electrode plate of an electrochemical device according to this application.

List of reference numerals:

1-first section
2-second section
3-third section
4-current collector

## DETAILED DESCRIPTION

Some embodiments of this application are described in detail below. No embodiment of this application is to be construed as a limitation on the protection scope of this application. Unless otherwise expressly specified, the following terms used herein have the meanings defined below.

The term "approximately" used herein is intended to describe and represent small variations. When used together with an event or situation, the term "approximately" may represent an example in which the event or situation occurs exactly or an example in which the event or situation occurs very approximately. For example, when used together with a numerical value, the term "approximately" may represent a variation range falling within ±10% of the numerical value, such as ±5%, ±4%, ±3%, ±2%, ±1%, ±0.5%, ±0.1%, or ±0.05% of the numerical value. In addition, a quantity, a ratio, or another numerical value herein is sometimes expressed in the format of a range. Understandably, such a range format is set out for convenience and brevity, and needs to be flexibly understood to include not only the numerical values explicitly specified and defined by the range, but also all individual numerical values or sub-ranges covered in the range as if each individual numerical value and each sub-range were explicitly specified.

In the detailed description of embodiments and claims, a list of items recited by using the term "one of" may mean any one of the recited items. For example, if items A and B are recited, the phrase "one of A and B" means A alone, or B alone. In another example, if items A, B, and C are recited, then the phrases "one of A, B, and C" and "one of A, B, or C" mean: A alone; B alone; or C alone. The item A may include a single element or a plurality of elements. The item B may include a single element or a plurality of elements. The item C may include a single element or a plurality of elements.

In the detailed description of embodiments and claims, a list of items recited by using the terms such as "at least one of" or "at least one type of" may mean any combination of the recited items. For example, if items A and B are recited, the phrases "at least one of A and B" and "at least one of A or B" mean: A alone; B alone; or both A and B. In another example, if items A, B, and C are recited, the phrases "at least one of A, B, and C" and "at least one of A, B, or C" mean: A alone; B alone; C alone; A and B (excluding C); A and C (excluding B); B and C (excluding A); or all of A, B, and C. The item A may include a single element or a plurality of elements. The item B may include a single element or a plurality of elements. The item C may include a single element or a plurality of elements.

In the detailed description of embodiments and claims, the number of carbon atoms is indicated by the number

following the capital letter "C", for example, "$C_1$ to $C_{10}$" and "$C_3$ to $C_{10}$". The number following the letter "C", for example,, "1", "3", or "10", represents the number of carbon atoms in a specific functional group. Such expressions indicate that the functional group may include 1 to 10 carbon atoms, and 3 to 10 carbon atoms, respectively. For example, "$C_1$ to $C_4$ alkyl" or "$C_{1-4}$ alkyl" means an alkyl group containing 1 to 4 carbon atoms, such as $CH_3$-, $CH_3CH_2$-, $CH_3CH_2CH_2$-, $(CH_3)_2CH$-, $CH_3CH_2CH_2CH_2$-, $CH_3CH_2CH(CH_3)$-, or $(CH_3)_3C$-.

As used herein, the term "alkyl" means a linear saturated hydrocarbon structure containing 1 to 10 carbon atoms. "Alkyl" also means a branched or cyclic hydrocarbon structure containing 3 to 10 carbon atoms. For example, an alkyl group may be an alkyl group containing 1 to 10 carbon atoms, an alkyl group containing 1 to 8 carbon atoms, an alkyl group containing 1 to 6 carbon atoms, or an alkyl group containing 1 to 4 carbon atoms. An alkyl containing a specified quantity of carbon atoms is intended to cover all geometric isomers containing the specified quantity of carbon atoms. Therefore, for example, "butyl" is intended to include n-butyl, sec-butyl, isobutyl, tert-butyl, and cyclobutyl; and "propyl" includes n-propyl, isopropyl, and cyclopropyl. Examples of alkyl include, but are not limited to, methyl, ethyl, n-propyl, isopropyl, cyclopropyl, n-butyl, isobutyl, sec-butyl, tert-butyl, cyclobutyl, n-pentyl, isopentyl, neopentyl, cyclopentyl, methylcyclopentyl, ethylcyclopentyl, n-hexyl, isohexyl, cyclohexyl, n-heptyl, octyl, cyclopropyl, cyclobutyl, norbomyl, and the like. In addition, the alkyl may be optionally substituted.

The term "alkenyl" means a linear or branched monovalent unsaturated hydrocarbon group containing at least one carbon-carbon double bond, and typically, 1, 2, or 3 carbon-carbon double bonds. Unless otherwise defined, an alkenyl generally contains 2 to 10 carbon atoms. For example, an alkenyl may be an alkenyl group containing 2 to 8 carbon atoms, an alkenyl group containing 2 to 6 carbon atoms, or an alkenyl group containing 2 to 4 carbon atoms. Representative alkenyls include, for example, vinyl, n-propenyl, isopropenyl, n-but-2-enyl, but-3-enyl, n-hex-3-enyl, and the like. In addition, the alkenyl may be optionally substituted.

The term "alkynyl" means a linear or branched monovalent unsaturated hydrocarbon group containing at least one carbon-carbon triple bond, and typically, 1, 2, or 3 carbon-carbon triple bonds. Unless otherwise defined, an alkynyl is generally an alkynyl group containing 2 to 10 carbon atoms, 2 to 8 carbon atoms, 2 to 6 carbon atoms, or 2 to 4 carbon atoms. Representative alkynyls include, for example, ethynyl, prop-2-ynyl (n-propynyl), n-but-2-ynyl, n-hex-3-ynyl, and the like. In addition, the alkynyl may be optionally substituted.

The term "aryl" covers a monocyclic system and a polycyclic system. Polycyclic may refer to two or more rings, of which two adjacent rings (which are "condensed") share two carbon atoms, at least one of which is aromatic, and the other of which may be, for example, a cycloalkyl group, a cycloalkenyl group, an aryl group, or a heterocyclic and/or heteroaryl group. For example, an aryl may be an aryl group containing 6 to 12 carbon atoms or 6 to 10 carbon atoms. Representative aryls include (for example), phenyl, methylphenyl, propylphenyl, isopropylphenyl, benzyl, naphth-1-yl, naphth-2-yl, and the like. In addition, the aryl may be optionally substituted.

Unless otherwise specified, substitution by the above groups means substitution by one or more halogens.

As used herein, the term "halogen" covers F, Cl, Br, and I, and is preferably F or Cl.

The term "positive active material" means a material that enables reversible intercalation and deintercalation of lithium ions. In some embodiments of this application, the positive active material includes, but is not limited to, a lithium-containing transition metal oxide.

## I. Electrochemical device

The electrochemical device according to this application may be any device in which an electrochemical reaction occurs. Specific examples of the electrochemical device include all kinds of primary batteries, secondary batteries, fuel batteries, solar batteries, or capacitors. In particular, the electrochemical device is a lithium secondary battery or a sodium secondary battery, including a lithium metal secondary battery, a lithium-ion secondary battery, a lithium polymer secondary battery, a lithium-ion polymer secondary battery, a sodium-ion secondary battery, a sodium polymer secondary battery, or a sodium-ion polymer secondary battery.

In some embodiments, the electrochemical device of this application includes a negative electrode and an electrolyte solution. The negative electrode includes a negative current collector and a negative electrode mixture layer disposed on at least one surface of the negative current collector. The negative electrode mixture layer includes a first section, a second section, and a third section arranged sequentially in a thickness direction. The third section is contiguous to the negative current collector. The second section is arranged between the first section and the third section in the thickness direction. The first section, the second section, and the third section are of an identical thickness. A ratio of a porosity P1 of the first section to a porosity P2 of the third section is P.

The electrolyte solution includes an additive A. The additive A is at least one selected from a compound of Formula I or a compound of Formula II:

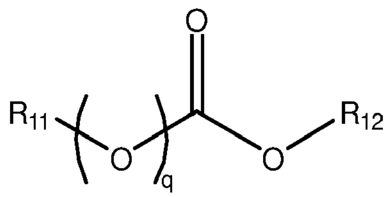
Formula I

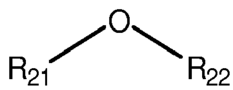
Formula II

In the formulas above, q is 0 or 1, at least one of $R_{11}$ or $R_{12}$ is selected from fluorine-substituted $C_1$ to $C_3$ alkyls, and $R_{21}$ and $R_{22}$ are selected from fluorine-substituted or unsubstituted $C_1$ to $C_4$ alkyls.
Based on the total mass of the electrolyte solution, an aggregate mass percent of the compound of Formula I and the compound of Formula II is w%, satisfying $1 \leq w \leq 60$.
The electrochemical device satisfies the following conditions: P = P1/P2, and $P/(w\% + 1) \geq 50\%$.
By introducing the additive A of a relatively low surface tension into the electrolyte solution, this application can reduce the surface tension of the electrolyte solution, accelerate infiltration of the electrolyte solution, and ensure that the electrolyte solution is distributed sufficiently and uniformly in regions of different porosities on the negative electrode mixture layer, thereby reducing polarization during charging of the electrochemical device and enhancing the charging capabilities.

**Positive electrode**

In some embodiments, the positive electrode includes a current collector and a positive electrode mixture layer located on the current collector. The positive electrode mixture layer includes a positive active material.
The positive active material includes at least one type of lithiated intercalation compound that enables reversible intercalation and deintercalation of lithium ions. In some embodiments of this application, the positive active material includes a lithium-containing transition metal oxide. In some embodiments, the positive active material includes a composite oxide. In some embodiments, the composite oxide contains lithium and at least one element selected from cobalt, manganese, or nickel.
In some embodiments, the positive active material may be coated with a coating layer on a surface thereof, or may be mixed with another compound coated with a coating layer. The coating layer may include at least one compound of a coating element, and the compound of a coating element is selected from: an oxide of a coating element, a hydroxide of a coating element, an oxyhydroxide of a coating element, an oxycarbonate of a coating element, and a hydroxycarbonate of a coating element. The compound included in the coating layer may be non-crystalline or crystalline.
In some embodiments, the coating element contained in the coating layer may include Mg, Al, Co, K, Na, Ca, Si, Ti, V, Sn, Ge, Ga, B, As, Zr, or any combination thereof. The coating may be applied by any method as long as the method does not adversely affect performance of the positive active material. For example, the method may include any coating method well known in the art, such as spraying and infiltrating.
The positive electrode mixture layer further includes a binder, and optionally includes a conductive material. The binder improves bonding between particles of the positive active material and bonding between the positive active material and a current collector.
In some embodiments, the binder includes, but is not limited to: polyvinyl alcohol, hydroxypropyl cellulose, diacetyl cellulose, polyvinyl chloride, carboxylated polyvinyl chloride, polyvinyl fluoride, a polymer containing ethylene oxide, polyvinylpyrrolidone, polyurethane, polytetrafluoroethylene, poly (1,1-difluoroethylene), polyethylene, polypropylene, styrene-butadiene rubber, acrylic styrene-butadiene rubber, epoxy resin, nylon, or the like.
In some embodiments, the conductive material includes, but is not limited to, a carbon-based material, a metal-based material, a conductive polymer, or a mixture thereof. In some embodiments, the carbon-based material is selected from natural graphite, artificial graphite, carbon black, acetylene black, Ketjen black, carbon fiber, or any combination thereof. In some embodiments, the metal-based material is selected from metal powder, metal fiber, copper, nickel, aluminum, or silver. In some embodiments, the conductive polymer is a polyphenylene derivative.
In some embodiments, the current collector may be, but is not limited to, aluminum.
The positive electrode may be prepared according to a preparation method known in the art. For example, the positive electrode may be obtained by the following method: mixing an active material, a conductive material, and a binder in a solvent to prepare an active material slurry, and coating the current collector with the active material slurry. In some embodiments, the solvent may include, is not limited to, N-methyl-pyrrolidone.
In some embodiments, the positive electrode is made of a positive electrode material formed by applying a positive electrode mixture layer onto a current collector, where the positive electrode mixture layer includes a lithium-containing transition metal-based compound powder and a binder.
In some embodiments, the positive electrode mixture layer is generally made by performing the following

operations: dry-mixing the positive electrode material and the binder (as necessary, in addition to a conductive material and a thickener) and making the mixture into a sheet, and crimping the obtained sheet onto the positive current collector; or, dissolving or dispersing such materials into a liquid medium to form a slurry, coating the positive current collector with the slurry, and drying the slurry.

**Negative electrode**

The negative electrode used in the electrochemical device of this application includes a negative current collector and a negative electrode mixture layer disposed on the negative current collector. As shown in Figure, the negative electrode mixture layer includes a first section, a second section, and a third section arranged sequentially in a thickness direction. The third section is contiguous to the negative current collector. The second section is arranged between the first section and the third section in the thickness direction. The first section, the second section, and the third section are of an identical thickness. A ratio of a porosity P1 of the first section to a porosity P2 of the third section is P.

In some embodiments, $P1 \geq 0.8P2$.

In some embodiments, $P = P1/P2$, and $P/(w\% + 1) \geq 50\%$.

In some embodiments, the first section, the second section, and the third section contain the same negative active material.

In some embodiments, the first section, the second section, and the third section exist in the same negative electrode mixture layer.

In some embodiments, a true density TD1 of the first section and a true density TD2 of the third section satisfy: $TD1 \leq TD2$.

In some embodiments, the true density TD1 of the first section and the true density TD2 of the third section satisfy: TD1 = TD2, and the first section and the third section are of identical composition.

The negative active material includes a material that enables reversible intercalation and deintercalation of lithium ions. In some embodiments, the material that enables reversible intercalation and deintercalation of lithium ions includes a carbon material. In some embodiments, the carbon material may be any carbon-based negative active material typically used in a lithium-ion rechargeable battery. In some embodiments, the carbon material includes, but is not limited to, crystalline carbon, non-crystalline carbon, and a mixture thereof. The crystalline carbon may be amorphous or flake-shaped, mini-flake-shaped, spherical or fibrous natural graphite or artificial graphite. The non-crystalline carbon may be soft carbon, hard carbon, mesophase pitch carbide, calcined coke, and the like.

In some embodiments, the negative electrode mixture layer includes a negative active material. The specific type of the negative active material is not limited, and may be selected as required. In some embodiments, the negative active material includes, but is not limited to, lithium metal, structured lithium metal, natural graphite, artificial graphite, mesocarbon microbeads (MCMB), hard carbon, soft carbon, silicon, a silicon-carbon composite, Li-Sn alloy, Li-Sn-O alloy, Sn, SnO, $SnO_2$, spinel-structured lithiated $TiO_2$-$Li_4Ti_5O_{12}$, Li-Al alloy, or any combination thereof. The silicon-carbon composite means silicon-carbon-based negative active material in which the mass of silicon is at least approximately 5 wt%.

In some embodiments, the negative active material includes at least one of artificial graphite, natural graphite, hard carbon, soft carbon, silicon alloy, or an oxide of silicon.

When the negative electrode includes a silicon-carbon compound, based on the total mass of the negative active material, the mass ratio between silicon and carbon is approximately 1: 10 to 10: 1. A volume median diameter $Dv_{50}$ of the silicon-carbon compound is approximately 0.1 $\mu$m to 20 $\mu$m. When the negative electrode includes an alloy material, the negative electrode mixture layer may be formed by using a method such as an evaporation method, a sputtering method, or a plating method. When the negative electrode includes metallic lithium, the negative electrode mixture layer is formed by a spherical skein-like conductive framework and metal particles dispersed in the conductive framework, for example. In some embodiments, a porosity of the spherical skein-like conductive framework is approximately 5% to approximately 85%. In some embodiments, a protection layer may be further disposed on the lithium-metal negative electrode mixture layer.

In some embodiments, the negative electrode mixture layer may include a binder, and optionally include a conductive material. The binder strengthens bonding between particles of the negative active material and bonding between the negative active material and the current collector. In some embodiments, the binder includes, but is not limited to: polyvinyl alcohol, carboxymethyl cellulose, hydroxypropyl cellulose, diacetyl cellulose, polyvinyl chloride, carboxylated polyvinyl chloride, polyvinyl fluoride, a polymer containing ethylene oxide, polyvinylpyrrolidone, polyurethane, polytetrafluoroethylene, poly(1,1-difluoroethylene), polyethylene, polypropylene, styrene-butadiene rubber, acrylic styrene-butadiene rubber, epoxy resin, nylon, or the like.

In some embodiments, the conductive material includes, but is not limited to, a carbon-based material, a metal-based material, a conductive polymer, or a mixture thereof. In some embodiments, the carbon-based material is selected from natural graphite, artificial graphite, carbon black, acetylene black, Ketjen black, carbon fiber, or any combination

thereof. In some embodiments, the metal-based material is selected from metal powder, metal fiber, copper, nickel, aluminum, or silver. In some embodiments, the conductive polymer is a polyphenylene derivative.

In some embodiments, the current collector includes, but is not limited to: a copper foil, a nickel foil, a stainless steel foil, a titanium foil, foamed nickel, foamed copper, a conductive-metal-clad polymer substrate, or any combination thereof.

The negative electrode may be prepared by a preparation method well known in the art. For example, the negative electrode may be obtained by the following method: mixing an active material, a conductive material, and a binder in a solvent to prepare an active material slurry, and coating the current collector with the active material slurry. In some embodiments, the solvent may include, but is not limited to, water or the like.

**Separator**

In some embodiments, the electrochemical device according to this application has a separator disposed between the positive electrode and the negative electrode to prevent short circuit. The material and the shape of the separator used in the electrochemical device in this application are not particularly limited, and the separator may be based on any technology disclosed in the prior art. In some embodiments, the separator includes a polymer or an inorganic compound or the like formed from a material that is stable to the electrolyte solution disclosed in this application.

For example, the separator may include a substrate layer and a surface treatment layer. The substrate layer is a non-woven fabric, film or composite film, which, in each case, is of a porous structure. The material of the substrate layer is at least one selected from polyethylene, polypropylene, polyethylene terephthalate, and polyimide. Specifically, the material of the substrate layer may be a polypropylene porous film, a polyethylene porous film, a polypropylene non-woven fabric, a polyethylene non-woven fabric, or a polypropylene-polyethylene-polypropylene porous composite film. There may be one or more substrate layers. When there are a plurality of substrate layers, the polymer composition in one substrate layer may be the same as or different from that in another substrate layer, and the weight-average molecular weight of the polymers is not completely identical between different substrate layers. When there are a plurality of substrate layers, the closed-pore temperature of polymers varies between different substrate layers.

In some embodiments, a surface treatment layer is disposed on at least one surface of the substrate layer. The surface treatment layer may be a polymer layer or an inorganic compound layer, or a layer compounded of a polymer and an inorganic compound.

In some embodiments, the separator includes a porous substrate and a coating layer. The coating layer includes inorganic particles and a binder.

In some embodiments, the thickness of the coating layer is approximately 0.5 $\mu m$ to approximately 10 $\mu m$, approximately 1 $\mu m$ to approximately 8 $\mu m$, or approximately 3 $\mu m$ to approximately 5 $\mu m$.

In some embodiments, the inorganic particles are at least one selected from $SiO_2$, $Al_2O_3$, CaO, $TiO_2$, $ZnO_2$, MgO, $ZrO_2$, $SnO_2$, $Al(OH)_3$, or AlOOH. In some embodiments, the diameter of the inorganic particles is approximately 0.001 $\mu m$ to approximately 3 $\mu m$.

In some embodiments, the binder is at least one selected from polyvinylidene fluoride (PVDF), poly(vinylidene fluoride-co-hexafluoroethylene) (PVDF-HFP), polyvinylpyrrolidone (PVP), polyacrylate, a pure acrylic emulsion (anionic acrylic emulsion copolymerized from acrylate and special functional monomers), a styrene acrylate emulsion (SAE, which is emulsified and copolymerized from styrene and acrylate monomers), or a styrene-butadiene emulsion (SBR, which is copolymerized from butadiene and styrene emulsions).

**Electrolyte solution**

In some embodiments, the electrolyte solution includes a lithium salt, an organic solvent, and an additive A. The additive A is at least one selected from a compound of Formula I or a compound of Formula II:

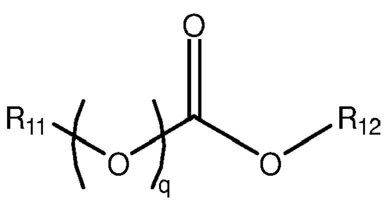 Formula I 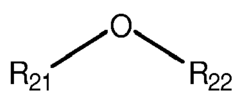 Formula II

In the formulas above, q is 0 or 1, at least one of $R_{11}$ or $R_{12}$ is selected from fluorine-substituted $C_1$ to $C_3$ alkyls, and $R_{21}$ and $R_{22}$ are selected from fluorine-substituted or unsubstituted $C_1$ to $C_4$ alkyls.

Based on the total mass of the electrolyte solution, a mass percent of the additive A is w%, satisfying $1 \leq w \leq 60$.

The electrochemical device satisfies the following conditions: P = P1/P2, and P/(w% + 1) ≥ 50%.

In some embodiments, the compound of Formula I includes at least one of the following compounds:

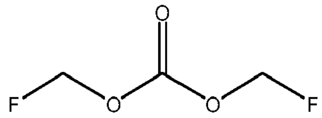

Formula I-1

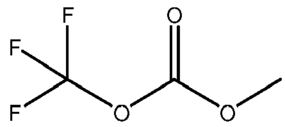

Formula I-2

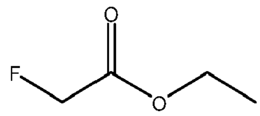,

Formula I-3

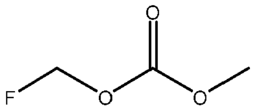

Formula I-4

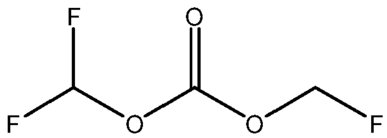

Formula I-5

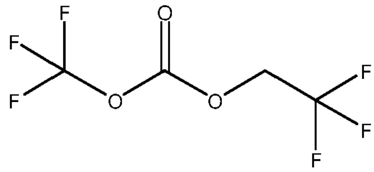,

Formula I-6

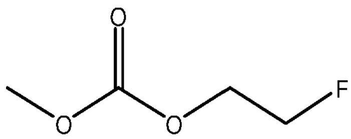

Formula I-7

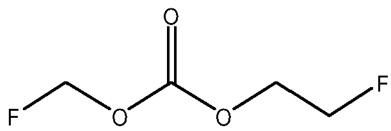

Formula I-8

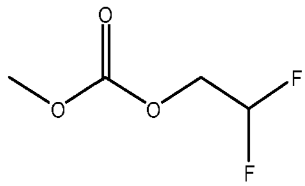,

Formula I-9

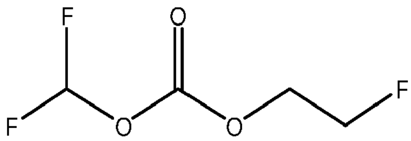

Formula I-10

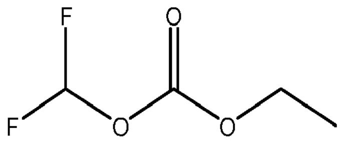

Formula I-11

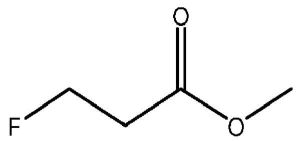,

Formula I-12

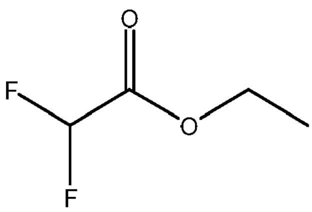

Formula I-13

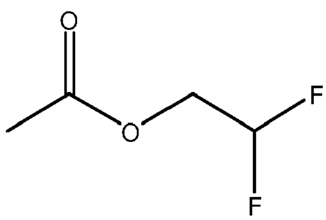

Formula I-14

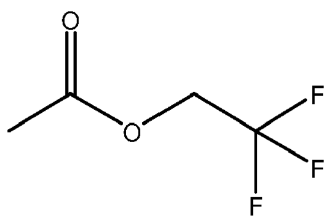,

Formula I-15

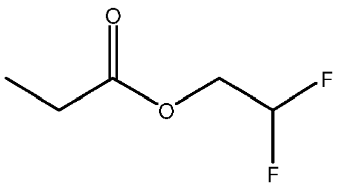 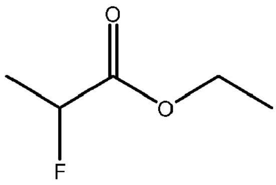 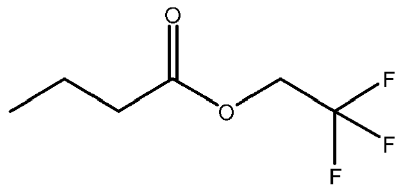 ,

Formula I-16 Formula I-17 Formula I-18

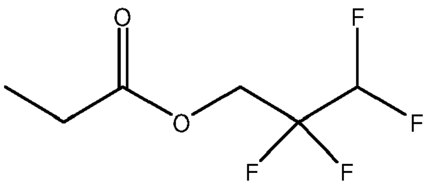 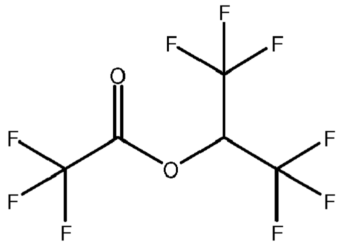 .

Formula I-19 Formula I-20

In some embodiments, the compound of Formula II includes at least one of the following compounds:

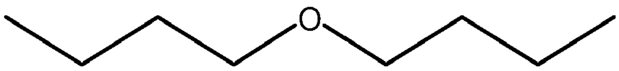 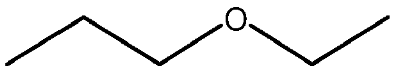 ,

Formula II-1 Formula II-2

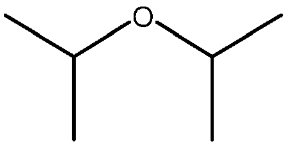 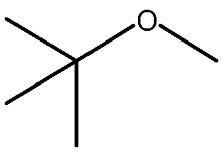 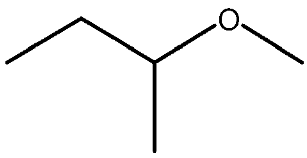 ,

Formula II-3 Formula II-4 Formula II-5

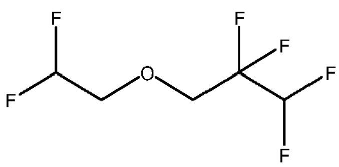 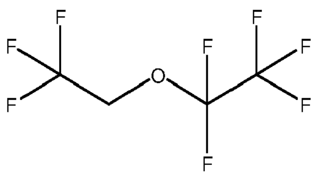 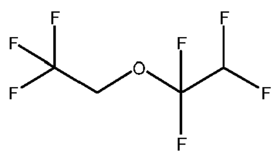 ,

Formula II-6 Formula II-7 Formula II-8

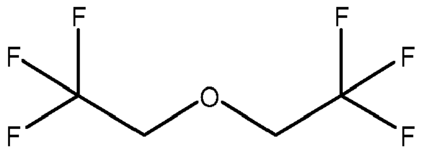

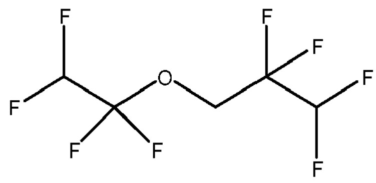
.

Formula II-9 Formula II-10

In some embodiments, $3 \leq w \leq 50$.

In some embodiments, $60\% \leq P/(w\% + 1) \leq 330\%$.

In some embodiments, $3 \leq w \leq 45$.

In some embodiments, $60\% \leq P/(w\% + 1) \leq 230\%$.

In some embodiments, $P1 \geq 0.8P2$.

In some embodiments, P1 is 20% to 50%.

In some embodiments, the electrolyte solution further includes an additive B. The additive B is at least one selected from a compound of Formula III or a nitrile additive:

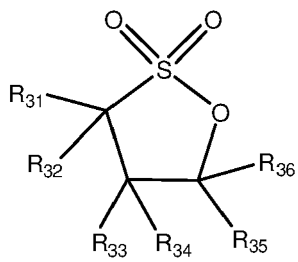
Formula III.

In Formula III above, $R_{31}$, $R_{32}$, $R_{33}$, $R_{34}$, $R_{35}$, and $R_{36}$ each are independently selected from H, a halogen atom, a halogen-substituted or unsubstituted C1 to C6 alkyl, or a C2 to C6 alkenyl or alkynyl.

The nitrile additive includes one of succinonitrile (SN), glutaronitrile (GN), adiponitrile (ADN), pimelonitrile, suberonitrile (SUN), sebaconitrile, azelonitrile, methyl glutaronitrile (m-GN), 1,3,6-hexanetricarbonitrile (HTCN), or trans-hexene dinitrile (DCB).

In some embodiments, based on the total mass of the electrolyte solution, a mass percent of the additive B is a%, satisfying $0.1 \leq a \leq 6$. In some embodiments, the additive B is a compound of Formula III.

In some embodiments, the additive B is a nitrile additive.

In some embodiments, the additive B is a combination of the compound of Formula III and the nitrile additive.

In some embodiments, the compound of Formula III includes at least one of the following compounds:

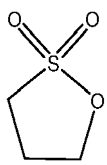
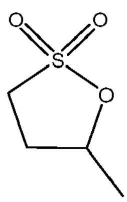
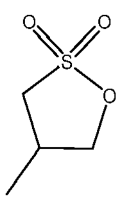
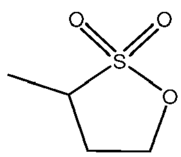
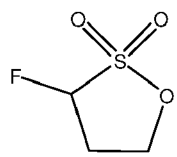
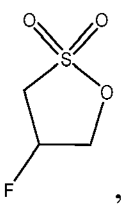
,

Formula III-1 Formula III-2 Formula III-3 Formula III-4 Formula III-5 Formula III-6

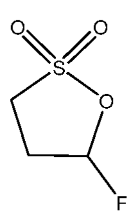

Formula III-7

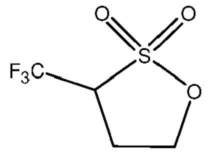

Formula III-8

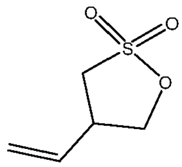

Formula III-9

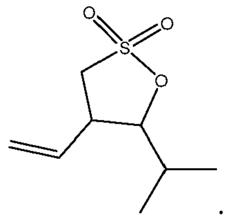

Formula III-10

The compound of Formula III can form a protection layer such as $Li_2SO_3$ on the surface of the positive electrode. The nitrile additive is oxidation-resistant to a relatively high degree, and can complex with the transition metal element on the surface of the positive electrode. In this way, the additive B used in the electrolyte solution can effectively form a protection layer on the surface of the positive electrode, thereby avoiding a side reaction of an ester solvent or ether solvent (such as the compound of Formula I and the compound of Formula II) on the positive electrode or negative electrode, and improving the high-temperature performance of the battery.

In some embodiments, the electrolyte solution further includes an additive C. The additive C is a surfactant. A hydrophilic-lipophilic balance value of the additive C is 0 to 15. Based on the total mass of the electrolyte solution, a mass percent of the additive C is b%, satisfying: $0.01 \leq b \leq 1$. Because the additive C includes amphiphilic groups that are both hydrophilic and lipophilic, the additive C can further reduce the surface tension of the electrolyte solution on the electrode surface and accelerate the infiltration of the electrolyte solution in the voids of the electrode coating layer.

In some embodiments, the additive C includes at least one of the following compounds: C1: $C_8F_{17}$-$SO_2N(C_3H_7)$-$(CH_2CH_2)_{20}$-$SO_3Li$; C2: lithium perfluorooctyl(trifluoromethyl-sulfonyl)imide; C3: lithium bis(nonafluorobutyl-sulfonyl)imide; C4: lithium (nonafluorobutyl-trifluoromethyl-sulfonyl)imide; C5: poly(oxyethylene)$_8$octylphenyl ether HO-$(CH_2CH_2O)_8$-Ph-$C_8H_{17}$; or C6: poly(oxyethylene)$_{20}$octadecyl ether HO-$(CH_2CH_2O)_{20}$-O-$C_{18}H_{37}$.

In some embodiments, the lithium salt is one or more of an inorganic lithium salt or an organic lithium salt. In some embodiments, the lithium salt contains at least one of fluorine, boron, or phosphorus. In some embodiments, the lithium salt is selected from one or more of: lithium hexafluorophosphate $LiPF_6$, lithium bis(trifluoromethanesulfonyl)imide $LiN(CF_3SO_2)_2$ (LiTFSI for short), lithium bis(fluorosulfonyl)imide $Li(N(SO_2F)_2$ (LiFSI for short), lithium hexafluoroarsenate $LiAsF_6$, lithium perchlorate $LiClO_4$, or lithium trifluoromethanesulfonate $LiCF_3SO_3$.

In some embodiments, a concentration of the lithium salt is 0.3 mol/L to 1.5 mol/L. In some embodiments, the concentration of the lithium salt is 0.5 mol/L to 1.3 mol/L or approximately 0.8 mol/L to 1.2 mol/L. In some embodiments, the concentration of the lithium salt is 1.10 mol/L.

The organic solvent includes at least one of ethylene carbonate (EC), propylene carbonate (PC), diethyl carbonate (DEC), ethyl methyl carbonate (EMC), dimethyl carbonate (DMC), sulfolane (SF), γ-butyrolactone (γ-BL), ethyl propyl carbonate, methyl formate (MF), ethyl formate (MA), ethyl acetate (EA), ethyl propionate (EP), propyl propionate (PP), methyl propionate, methyl butyrate, ethyl butyrate, ethyl methyl fluorocarbonate, dimethyl fluorocarbonate, or diethyl fluorocarbonate.

In some embodiments, the mass percent of the solvent is 70 wt% to 95 wt% of the total mass of the electrolyte solution.

In some embodiments, the organic solvent may be a carbonate compound, a carboxylate compound, an ether compound, another organic solvent, or any combination thereof. The carbonate compound may be a chain carbonate compound, a cyclic carbonate compound, a fluorocarbonate compound, or any combination thereof. Examples of the chain carbonate compound are dimethyl carbonate (DMC), diethyl carbonate (DEC), dipropyl carbonate (DPC), methyl propyl carbonate (MPC), ethylene propyl carbonate (EPC), ethyl methyl carbonate (EMC), or any combination thereof. Examples of the cyclic carbonate compound are ethylene carbonate (EC), propylene carbonate (PC), butylene carbonate (BC), vinyl ethylene carbonate (VEC), or any combination thereof. Examples of the fluorocarbonate compound are fluoroethylene carbonate (FEC), 1, 2-difluoroethylene carbonate, 1,1-difluoroethylene carbonate, 1,1,2-trifluoroethylene carbonate, 1,1,2,2-tetrafluoroethylene carbonate, 1-fluoro-2-methyl ethylene, 1-fluoro-1-methyl ethylene carbonate, 1,2-difluoro-1-methyl ethylene carbonate, 1,1,2-trifluoro-2-methyl ethylene carbonate, trifluoromethyl ethylene carbonate, or any combination thereof. Examples of the carboxylate compound are methyl formate, methyl acetate, ethyl acetate, n-propyl acetate, tert-butyl acetate, methyl propionate, ethyl propionate, propyl propionate, γ-butyrolactone, decanolactone, valerolactone, mevalonolactone, caprolactone, or any combination thereof. Examples of the ether compound are dibutyl ether, tetraglyme, diglyme, 1,2-dimethoxyethane, 1,2-diethoxyethane, ethoxy-methoxyethane, 2-methyltetrahydrofuran, tetrahydrofuran, or any combination thereof.

In some embodiments, examples of the other organic solvent are dimethyl sulfoxide, 1,2-dioxolane, sulfolane, methyl sulfolane, 1,3-dimethyl-2-imidazolidinone, N-methyl-2-pyrrolidone, formamide, dimethylformamide, acetonitrile,

trimethyl phosphate, triethyl phosphate, trioctyl phosphate, phosphate ester, or any combination thereof.

The electrolyte solution used in the electrochemical device of this application is any of the electrolyte solutions described herein above. In addition, the electrolyte solution used in the electrochemical device of this application may further include other electrolyte solutions obtained without departing from the spirit and scope of this application.

## II. Electronic device

The electronic device of this application may be any device that uses the electrochemical device disclosed herein.

In some embodiments, the electronic device includes, but is not limited to, a notebook computer, pen-inputting computer, mobile computer, e-book player, portable phone, portable fax machine, portable photocopier, portable printer, stereo headset, video recorder, liquid crystal display television set, handheld cleaner, portable CD player, mini CD-ROM, transceiver, electronic notepad, calculator, memory card, portable voice recorder, radio, backup power supply, motor, automobile, motorcycle, power-assisted bicycle, bicycle, lighting appliance, toy, game console, watch, electric tool, flashlight, camera, large household battery, lithium-ion capacitor, or the like.

To fulfill the above objectives and enable a person skilled in the art to understand the technical solutions hereof, the technical solutions of this application are described below by using the following examples. It is hereby noted that the described embodiments are merely a part of but not all of the embodiments of this application.

## III. Test methods

1. Testing the charge time of a lithium-ion battery

Putting the lithium-ion battery into a 25 °C thermostat, and leaving the battery to stand for 30 minutes so that the temperature of the lithium-ion battery is constant. Charging the constant-temperature lithium-ion battery at a constant current of 3 C until the voltage reaches 4.3 V, and then charging the battery at a constant current of 1 C until the voltage reaches 4.35 V, and then charging the battery at a constant voltage until the current reaches 0.05 C. Recording the charge time in this process as T.

2. Testing the low-temperature discharge capacity retention rate of the lithium-ion battery

Putting a lithium-ion battery into a temperature-adjustable high and low-temperature chamber. Setting the temperature to 25 °C, and leaving the battery to stand for 60 minutes. Subsequently, discharging the battery at a current of 0.2 C until the voltage reaches 3.0 V, and then leaving the battery to stand for 15 minutes. Charging the battery at a constant current of 3 C until the voltage reaches 4.3 V, and then charging the battery at a constant current of 1 C until the voltage reaches 4.35 V, charging the battery at a constant voltage until the current reaches 0.05 C, and leaving the battery to stand for 15 minutes. Subsequently, discharging the battery at a current of 0.2 C until the voltage reaches 3.0 V. Recording the discharge capacity in this step as $C_{25}$. Adjusting the temperature of the high and low-temperature chamber to -20 °C, repeating the above charge and discharge process, and recording the discharge capacity as $C_{-20}$.

Calculating the low-temperature discharge capacity retention rate as: $C = C_{-20}/C_{25}$.

3. Testing the high-temperature storage performance of the lithium-ion battery

Charging, in a constant-temperature room, a lithium-ion battery at a constant current of 3 C until the voltage reaches 4.3 V, and then charging the battery at a constant current of 1 C until the voltage reaches 4.35 V, and then charging the battery at a constant voltage until the current reaches 0.05 C. Recording the battery thickness at this time as $d_1$. Subsequently, putting the lithium-ion battery into a muffle oven in which the temperature is 60 °C, keeping the battery in the muffle oven for 7 days, and then taking out the battery and measuring the thickness $d_2$ of the battery at this time immediately. Calculating the high-temperature storage thickness expansion rate as: $d = (d_2 - d_1)/d_1$.

4. Characterizing a two-dimensional porosity of an electrode plate

Take a cold-pressed electrode plate or an electrode plate obtained by dissembling a battery cell, and obtaining a cross-section of the electrode plate by means of ion beam cross-section polishing, as shown in Figure. Subsequently, capture an SEM image by using a scanning electron microscope (SEM), and then determining the voids and an active material region through an image segmentation algorithm. Obtaining the porosity of the corresponding region based on the percentage of the area of the voids.

**Embodiments 1-1 to 1-24**

Preparing a negative electrode: Mixing artificial graphite as a negative active material, sodium carboxymethyl cellulose (CMC) as a thickener, and styrene butadiene rubber (SBR) as a binder at a mass ratio of 97: 1: 2. Adding deionized water, and stirring the mixture with a vacuum mixer to obtain a negative electrode slurry in which a solid content is 54 wt%. Coating a negative current collector copper foil with the negative electrode slurry evenly, drying the coated copper foil at 85 °C, and performing cold pressing, cutting, and slitting. Drying the electrode plate for 12 hours in a 120 °C vacuum environment to obtain a negative electrode plate.

Preparing a positive electrode: Mixing $LiNi_{0.5}Co_{0.2}Mn_{0.3}O_2$ as a positive active material, Super P as a conductive agent, and polyvinylidene difluoride as a binder at a mass ratio of 97: 1.4: 1.6. Adding N-methyl-pyrrolidone (NMP), and stirring the mixture with a vacuum blender until a homogeneous state, so as to obtain a positive electrode slurry in which a solid content is 72 wt%. Coating a positive current collector aluminum foil with the positive electrode slurry evenly, drying the coated aluminum foil at 85 °C, and performing cold pressing, cutting, and slitting, and then drying the electrode plate for 4 hours in an 85 °C vacuum environment to obtain a positive electrode plate.

Preparing an electrolyte solution: Formulating an electrolyte solution that contains a lithium salt $LiPF_6$ at a concentration of 1 mol/L in the following process: Weighing out 12.5 wt% $LiPF_6$ in a dry argon atmosphere glovebox, and then weighing out ethylene carbonate (EC) at a mass percent of 20 wt%, a constituent A at a mass percent of w% (referring to Table 1), diethyl carbonate (DEC) at a mass percent of (1-12.5%-20%-w%). Mixing, dissolving and stirring the constituents well, and adding the lithium salt $LiPF_6$ weighed out in advance. Mixing the constituents well to obtain an electrolyte solution.

Preparing a separator: Using a 9 μm-thick polyethylene (PE) film as a separator, coating the film with a polyvinylidene difluoride (PVDF) slurry and an $Al_2O_3$ slurry, and drying the slurry to obtain a final separator.

Assembling a lithium-ion battery: Stacking the positive electrode, the separator, and the negative electrode sequentially, placing the separator between positive electrode plate and the negative electrode plate, winding the stacked structure, and welding tabs to obtain a bare cell. Putting the bare cell into an aluminum laminated film used as outer package foil. Injecting the above electrolyte solution. Performing steps such as vacuum sealing, standing, chemical formation, shaping, and capacity test to obtain a pouch-type lithium-ion battery of 3.3 mm × 39 mm × 96 mm in size.

**Comparative Embodiments 1-1 to 1-3**

Comparative Embodiments 1-1 to 1-3 are performed with reference to Embodiment 1-1, and the additives used in Comparative Embodiments 1-1 to 1-3 are shown in Table 1.

**Table 1**

| Embodiment | P1 | P2 | Additive A and content w% thereof (wt%) | P/(w%+1) | Charge time T (min) | Low-temperature discharge capacity retention rate C |
|---|---|---|---|---|---|---|
| Comparative Embodiment 1-1 | 30.0% | 31.2% | \ | 96.2% | 27.0 | 76.8% |
| Comparative Embodiment 1-2 | 26.8% | 33.5% | \ | 80.0% | 28.0 | 75.8% |
| Comparative Embodiment 1-3 | 26.8% | 33.5% | 5% Formula I-6 | 76.2% | 27.8 | 76.2% |
| Embodiment 1-1 | 30.0% | 31.2% | 1% Formula I-6 | 95.2% | 25.0 | 85.0% |
| Embodiment 1-2 | 30.0% | 31.2% | 3% Formula I-6 | 93.4% | 24.2 | 85.4% |
| Embodiment 1-3 | 30.0% | 31.2% | 5% Formula I-6 | 91.6% | 24.3 | 85.5% |
| Embodiment 1-4 | 30.0% | 31.2% | 10% Formula I-6 | 87.4% | 24.1 | 86.1% |
| Embodiment 1-5 | 30.0% | 31.2% | 30% Formula I-6 | 74.0% | 23.2 | 86.5% |
| Embodiment 1-6 | 30.0% | 31.2% | 45% Formula I-6 | 66.3% | 23.1 | 86.6% |
| Embodiment 1-7 | 30.0% | 31.2% | 50% Formula I-6 | 64.1% | 23.7 | 86.4% |
| Embodiment 1-8 | 30.0% | 31.2% | 60% Formula I-6 | 60.1% | 24.3 | 86.2% |
| Embodiment 1-9 | 30.0% | 31.2% | 5% Formula I-2 | 91.6% | 24.3 | 85.9% |

(continued)

| Embodiment | P1 | P2 | Additive A and content w% thereof (wt%) | P/ (w%+1) | Charge time T (min) | Low-temperature discharge capacity retention rate C |
|---|---|---|---|---|---|---|
| Embodiment 1-10 | 30.0% | 31.2% | 5% Formula I-5 | 91.6% | 24.4 | 85.8% |
| Embodiment 1-11 | 30.0% | 31.2% | 5% Formula I-14 | 91.6% | 24.1 | 86.2% |
| Embodiment 1-12 | 30.0% | 31.2% | 5% Formula I-18 | 91.6% | 24.2 | 85.1% |
| Embodiment 1-13 | 30.0% | 31.2% | 5% Formula II-2 | 91.6% | 24.7 | 86.0% |
| Embodiment 1-14 | 30.0% | 31.2% | 5% Formula II-6 | 91.6% | 24.5 | 85.6% |
| Embodiment 1-15 | 30.0% | 31.2% | 5% Formula II-10 | 91.6% | 24.6 | 85.5% |
| Embodiment 1-16 | 30.0% | 31.2% | 5% Formula I-14 + 1% Formula II-6 | 90.7% | 24.5 | 84.2% |
| Embodiment 1-17 | 20.2% | 24.5% | 5% Formula I-6 | 78.5% | 24.8 | 85.3% |
| Embodiment 1-18 | 33.1% | 30.2% | 5% Formula I-6 | 104.4% | 24.4 | 86.2% |
| Embodiment 1-19 | 35.0% | 35.0% | 5% Formula I-6 | 95.2% | 24.7 | 86.4% |
| Embodiment 1-20 | 42.0% | 35.0% | 5% Formula I-6 | 114.3% | 24.2 | 86.70% |
| Embodiment 1-21 | 50.0% | 30.2% | 5% Formula I-6 | 157.7% | 24.1 | 86.80% |
| Embodiment 1-22 | 20.0% | 25.0% | 60% Formula I-6 | 50.0% | 24.7 | 85.50% |
| Embodiment 1-23 | 50.0% | 15.0% | 45% Formula I-6 | 229.9% | 24.9 | 85.20% |
| Embodiment 1-24 | 50.0% | 15.0% | 1% Formula I-6 | 330.0% | 25.3 | 85.10% |

As can be seen from Embodiments 1-1 to 1-24 versus Comparative Embodiments 1-1 to 1-3, when P/(w%+1) falls within the specified range, the charge speed and the low-temperature discharge capacity retention rate of the lithium-ion battery can be increased. That is because the difference in porosity between the first section and the third section affects the infiltration effect of the electrolyte solution. The compound of Formula I or Formula II possesses relatively low surface tension, and can ensure that the pores in the first section of the electrode plate are uniformly filled with the electrolyte solution, thereby implementing fast charge and exhibiting high capabilities of low-temperature discharge.

**Embodiments 2-1 to 2-19**

Embodiments 2-1 to 2-19 are performed with reference to Embodiment 1-2. The additives used in Embodiments 2-1 to 2-19 are shown in Table 2, and the mass percent of the diethyl carbonate (DEC) is (1-12.5%-20%-w%-a%-b%).

**Table 2**

| | Additive B and content a% thereof (wt%) | Additive C and content b% thereof (wt%) | Charge time | High-temperature storage thickness expansion rate |
|---|---|---|---|---|
| Embodiment 1-2 | \ | \ | 24.2 | 21.50% |
| Embodiment 2-1 | 0.1% Formula III-1 | \ | 24.5 | 18.40% |
| Embodiment 2-2 | 1% Formula III-1 | \ | 24.6 | 15.9% |
| Embodiment 2-3 | 5 % Formula III-1 | \ | 24.8 | 11.7% |
| Embodiment 2-4 | 3% Formula III-4 | \ | 24.7 | 12.3% |

(continued)

| | Additive B and content a% thereof (wt%) | Additive C and content b% thereof (wt%) | Charge time | High-temperature storage thickness expansion rate |
|---|---|---|---|---|
| Embodiment 2-5 | 3% Formula III-7 | \ | 24.7 | 12.0% |
| Embodiment 2-6 | 0.3% Formula III-1 + 2% Formula III-4 | \ | 24.6 | 12.5% |
| Embodiment 2-7 | 1% SN | \ | 24.6 | 16.3% |
| Embodiment 2-8 | 1% ADN | \ | 24.6 | 15.8% |
| Embodiment 2-9 | 1% HTCN | \ | 24.8 | 13.8% |
| Embodiment 2-10 | 1% SUN | \ | 24.7 | 14.0% |
| Embodiment 2-11 | 1% m-GN | \ | 24.6 | 16.0% |
| Embodiment 2-12 | 1% ADN + 1% HTCN | \ | 24.8 | 13.1% |
| Embodiment 2-13 | 3% III-1 + 1% ADN | \ | 24.8 | 11.5% |
| Embodiment 2-14 | 2% Formula III-1 + 1% ADN + 1% HTCN | \ | 24.9 | 10.7% |
| Embodiment 2-15 | 2% Formula III-1 + 1% ADN + 1% HTCN | 0.01% C1 | 24.5 | 10.8% |
| Embodiment 2-16 | 2% Formula III-1 + 1% ADN + 1% HTCN | 0.1% C1 | 24.3 | 10.7% |
| Embodiment 2-17 | 2% Formula III-1 + 1% ADN + 1% HTCN | 1% C1 | 24.2 | 10.6% |
| Embodiment 2-18 | 2% Formula III-1 + 1% ADN + 2% HTCN | 0.1% C4 | 24.4 | 10.7% |
| Embodiment 2-19 | 2% Formula III-1 + 1% ADN + 3% HTCN | 0.1% C6 | 24.3 | 10.8% |

As can be seen from Embodiments 2-1 to 2-14 in Table 2, the Additive B can serve to further improve the high-temperature storage performance. That is because the Additive B can form a stable protection layer on the surface of the positive electrode, and work together with the element F in Formula I to optimize the structure of the positive electrode protection layer, thereby implementing stable protection. As can be seen from Embodiments 2-15 to 2-19 versus Embodiment 2-14, the Additive C can further enhance the charging capabilities and shorten the charge time without deteriorating the high-temperature storage performance. Because the additive C includes amphiphilic groups that are both hydrophilic and lipophilic, the additive C can further reduce the surface tension of the electrolyte solution on the electrode surface and accelerate the infiltration of the electrolyte solution in the voids of the electrode coating layer.

Although illustrative embodiments have been demonstrated and described above, a person skilled in the art understands that the foregoing embodiments are never to be construed as a limitation on this application, and any changes, replacements, and modifications may be made to the embodiments without departing from the spirit, principles, and scope of this application. Such changes, replacements, and modifications still fall within the protection scope of this application.

## Claims

1. An electrochemical device, comprising a negative electrode and an electrolyte solution, **characterized in that** the negative electrode comprises a negative current collector and a negative electrode mixture layer disposed on at least one surface of the negative current collector; the negative electrode mixture layer comprises a first section, a second section, and a third section arranged sequentially in a thickness direction; the third section is contiguous to the negative current collector; the second section is arranged between the first section and the third section in the thickness direction; the first section, the second section, and the third section are of an identical thickness; and a ratio of a porosity P1 of the first section to a porosity P2 of the third section is P;

   the electrolyte solution comprises an additive A, and the additive A is at least one selected from a compound of Formula I or a compound of Formula II:

   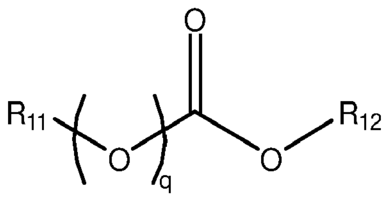 Formula I 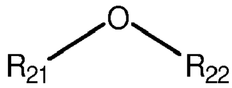 Formula II

   **characterized in that**, q is 0 or 1, at least one of $R_{11}$ or $R_{12}$ is selected from fluorine-substituted $C_1$ to $C_3$ alkyls, and $R_{21}$ and $R_{22}$ are selected from fluorine-substituted or unsubstituted $C_1$ to $C_4$ alkyls;
   based on a total mass of the electrolyte solution, a mass percent of the additive A is w%, and $1 \leq w \leq 60$; and

   $$P/(w\% + 1) \geq 50\%.$$

2. The electrochemical device according to claim 1, **characterized in that**, the compound of Formula I comprises at least one of the following compounds:

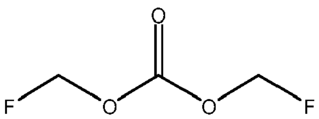

Formula I-1,

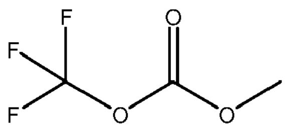

Formula I-2,

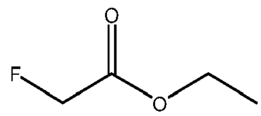

Formula I-3,

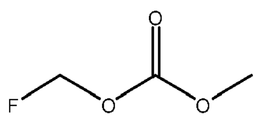

Formula I-4,

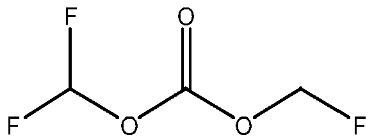

Formula I-5,

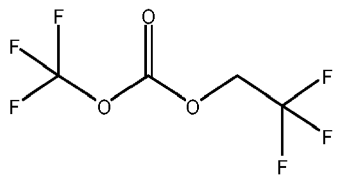

Formula I-6,

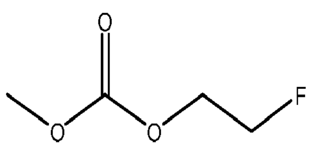

Formula I-7,

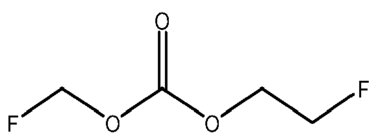

Formula I-8,

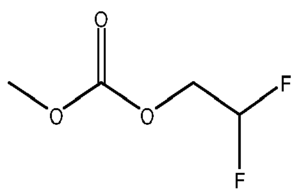

Formula I-9,

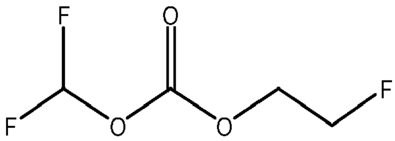

Formula I-10,

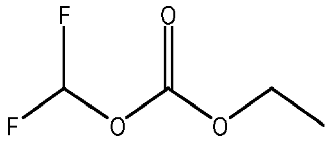

Formula I-11,

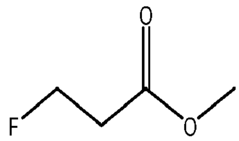

Formula I-12,

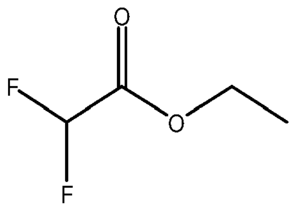

Formula I-13,

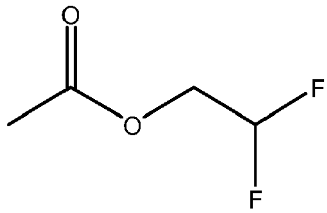

Formula I-14,

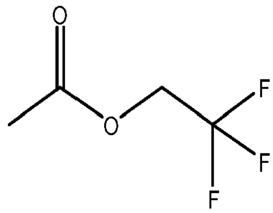

Formula I-15,

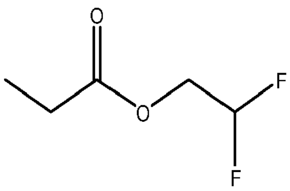

Formula I-16,

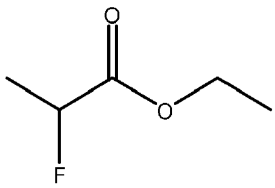

Formula I-17,

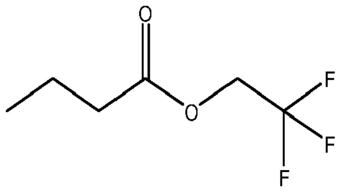

Formula I-18,

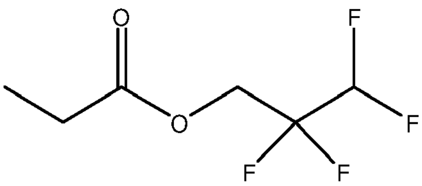

Formula I-19, or

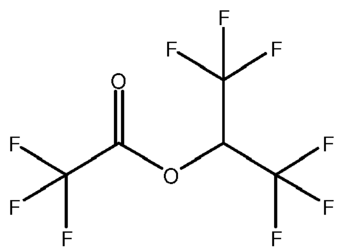

;

Formula I-20

and/or,
the compound of Formula II comprises at least one of the following compounds:

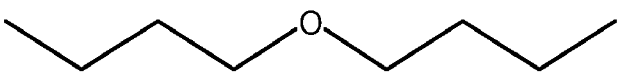

Formula II-1,

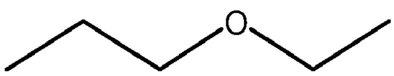

Formula II-2,

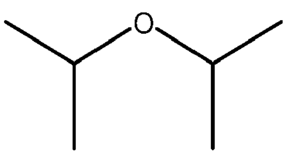

Formula II-3,

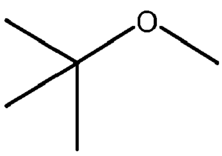

Formula II-4,

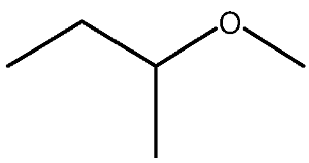

Formula II-5,

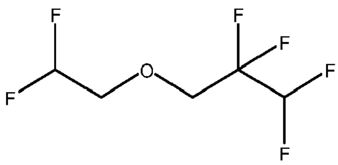

Formula II-6,

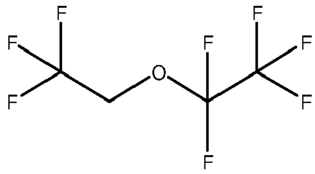

Formula II-7,

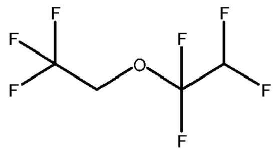

Formula II-8,

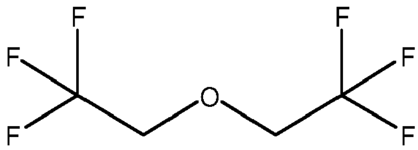

Formula II-9, or

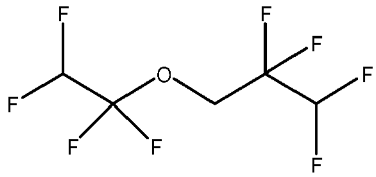

Formula II-10.

**3.** The electrochemical device according to claim 1, **characterized in that**, the electrochemical device satisfies at least one of the following conditions:

$$(1)\ 3 \leq w \leq 50;$$

or

$$(2)\ 60\% \leq P/(w\% + 1) \leq 330\%.$$

**4.** The electrochemical device according to claim 1, **characterized in that**, the electrochemical device satisfies at least one of the following conditions:

$$(3)\ 3 \leq w \leq 45;$$

or

$$(4)\ 60\% \leq P/(w\% + 1) \leq 230\%.$$

**5.** The electrochemical device according to claim 1, **characterized in that**, P1 ≥ 0.8P2.

**6.** The electrochemical device according to claim 1, **characterized in that**, P1 is 20% to 50%.

**7.** The electrochemical device according to claim 1, **characterized in that**, the electrolyte solution further comprises an additive B, and the additive B is at least one selected from a compound of Formula III or a nitrile additive:

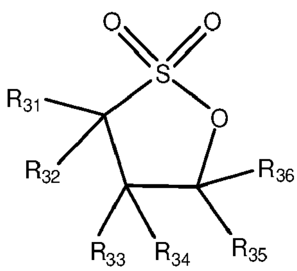

Formula III

wherein $R_{31}$, $R_{32}$, $R_{33}$, $R_{34}$, $R_{35}$, and $R_{36}$ each are independently selected from H, a halogen atom, a halogen-substituted or unsubstituted C1 to C6 alkyl, or a C2 to C6 alkenyl or alkynyl;
the nitrile additive comprises at least one selected from the group consisting of succinonitrile, glutaronitrile, adiponitrile, pimelonitrile, suberonitrile, sebaconitrile, azelonitrile, methyl glutaronitrile, 1,3,6-hexanetricarbonitrile, and trans-hexene dinitrile; and
based on the total mass of the electrolyte solution, a mass percent of the additive B is a%, satisfying $0.1 \le a \le 6$.

8. The electrochemical device according to claim 7, **characterized in that**, the compound of Formula III comprises at least one of the following compounds:

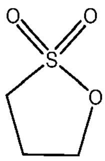 Formula III-1, 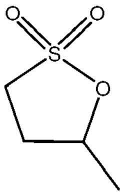 Formula III-2, 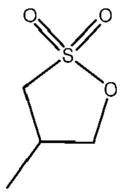 Formula III-3, 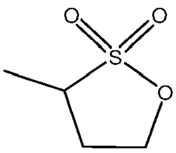 Formula III-4, 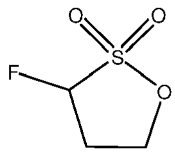 Formula III-5, 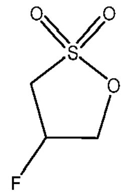 Formula III-6,

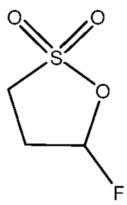 Formula III-7, 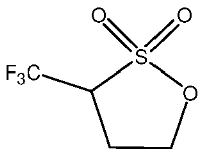 Formula III-8, 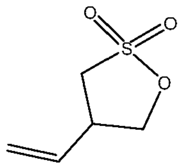 Formula III-9, 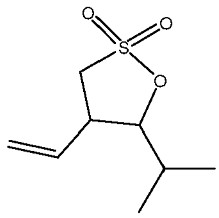 Formula III-10.

9. The electrochemical device according to claim 1, **characterized in that**, the electrolyte solution further comprises an additive C; a hydrophilic-lipophilic balance value of the additive C is 0 to 15; and, based on the total mass of the electrolyte solution, a mass percent of the additive C is b%, and $0.01 \le b \le 1$.

10. The electrochemical device according to claim 9, **characterized in that** the additive C comprises at least one of the following compounds:

C1: $C_8F_{17}$-$SO_2N(C_3H_7)$-$(CH_2CH_2)_{20}$-$SO_3Li$;
C2: lithium perfluorooctyl(trifluoromethyl-sulfonyl)imide;
C3: lithium bis(nonafluorobutyl-sulfonyl)imide;
C4: lithium (nonafluorobutyl-trifluoromethyl-sulfonyl)imide;
C5: poly(oxyethylene)$_8$octylphenyl ether HO-$(CH_2CH_2O)_8$-Ph-$C_8H_{17}$; or
C6: poly(oxyethylene)$_{20}$octadecyl ether HO-$(CH_2CH_2O)_{20}$-O-$C_{18}H_{37}$.

11. The electrochemical device according to claim 1, **characterized in that**, a true density TD1 of the first section and a true density TD2 of the third section satisfy: TD1 ≤ TD2.

12. The electrochemical device according to claim 11, **characterized in that**, TD1 = TD2, and the first section and the third section are of identical composition.

13. An electronic device, **characterized in that** the electronic device comprises the electrochemical device according to any one of claims 1 to 12.

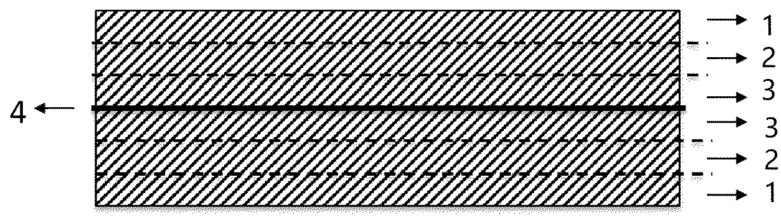


FIGURE

Europäisches Patentamt
European Patent Office
Office européen des brevets

# EUROPEAN SEARCH REPORT

Application Number
EP 24 16 6255

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2021/328268 A1 (WEN QIAN [CN] ET AL) 21 October 2021 (2021-10-21) * paragraphs [0069], [0143]; compounds F, G * ----- | 1-13 | INV. H01M4/133 H01M4/587 H01M10/0525 H01M10/0567 ADD. H01M4/02 |
| X | US 2020/099098 A1 (WANG KEFEI [CN] ET AL) 26 March 2020 (2020-03-26) * paragraph [0112]; table 3 * ----- | 1-13 | |

TECHNICAL FIELDS SEARCHED (IPC)

H01M

The present search report has been drawn up for all claims

1

| Place of search | Date of completion of the search | Examiner |
|---|---|---|
| Munich | 9 August 2024 | Steinreiber, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 16 6255

This annex lists the patent family members relating to the patent documents cited in the above-mentioned European search report. The members are as contained in the European Patent Office EDP file on
The European Patent Office is in no way liable for these particulars which are merely given for the purpose of information.

09-08-2024

| Patent document cited in search report | Publication date | Patent family member(s) | Publication date |
|---|---|---|---|
| US 2021328268 A1 | 21-10-2021 | CN 109935783 A | 25-06-2019 |
| | | EP 3928368 A1 | 29-12-2021 |
| | | US 2021328268 A1 | 21-10-2021 |
| | | WO 2020168764 A1 | 27-08-2020 |
| US 2020099098 A1 | 26-03-2020 | CN 109301326 A | 01-02-2019 |
| | | DK 3627606 T3 | 10-01-2022 |
| | | DK 3910714 T3 | 18-09-2023 |
| | | EP 3627606 A1 | 25-03-2020 |
| | | EP 3907801 A1 | 10-11-2021 |
| | | EP 3910714 A1 | 17-11-2021 |
| | | EP 4235900 A2 | 30-08-2023 |
| | | EP 4362162 A2 | 01-05-2024 |
| | | EP 4362163 A2 | 01-05-2024 |
| | | EP 4383396 A2 | 12-06-2024 |
| | | EP 4404322 A2 | 24-07-2024 |
| | | ES 2902422 T3 | 28-03-2022 |
| | | ES 2955407 T3 | 30-11-2023 |
| | | FI 3910714 T3 | 06-09-2023 |
| | | HU E063500 T2 | 28-01-2024 |
| | | PL 3627606 T3 | 07-03-2022 |
| | | PL 3910714 T3 | 15-04-2024 |
| | | US 2020099098 A1 | 26-03-2020 |
| | | US 2021028489 A1 | 28-01-2021 |
| | | US 2021028490 A1 | 28-01-2021 |
| | | US 2022407108 A1 | 22-12-2022 |
| | | US 2022407109 A1 | 22-12-2022 |
| | | US 2023102805 A1 | 30-03-2023 |
| | | US 2023128988 A1 | 27-04-2023 |
| | | US 2023335794 A1 | 19-10-2023 |
| | | US 2023352734 A1 | 02-11-2023 |
| | | US 2024250300 A1 | 25-07-2024 |
| | | US 2024258565 A1 | 01-08-2024 |
| | | US 2024258566 A1 | 01-08-2024 |
| | | US 2024266603 A1 | 08-08-2024 |

EPO FORM P0459

For more details about this annex : see Official Journal of the European Patent Office, No. 12/82